# EUROPEAN PATENT SPECIFICATION

(11) **EP 1 660 525 B1**
(45) Date of publication and mention of the grant of the patent: **29.11.2006**
(21) Application number: 04738163.7
(22) Date of filing: 23.08.2004
(51) Int. Cl.: C07K 14/415

(54) **PLANT-DERIVED PEPTIDES HARBORING WATER-CLEANING AND ANTIMICROBIAL ACTIVITIES**
AUS PFLANZEN GEWONNENE PEPTIDE MIT WASSERREINIGENDER UND ANTIMIKROBIELLER WIRKUNG
PEPTIDES DERIVES DE PLANTES AUX PROPRIETES DE NETTOYAGE D'EAU ET ANTIMICROBIENNES

(30) Priority: 22.08.2003 WO PCT/CH03/00568
(43) Date of publication of application: 31.05.2006
(73) Proprietor: Optima Environnement SA, 1260 Nyon (CH)
(72) Inventor: MERMOD, Nicolas, CH-1164 Buchillon (CH); SUAREZ, Mougli, CH-1010 Lausanne (CH)
(74) Representative: Grosfillier, Philippe
(86) International application number: PCT/CH2004/000536
(87) International publication number: WO 2005/019253

(56) References cited:
- WO-A2-03/008441
- SUAREZ M ET AL: "Expression of a plant-derived peptide harboring water-cleaning and antimicrobial activities." BIOTECHNOLOGY AND BIOENGINEERING, vol. 81, no. 1, 5 January 2003 (2003-01-05), pages 13-20, XP002315279 ISSN: 0006-3592
- GASSENSCHMIDT URSULA ET AL: "Isolation and characterization of a flocculating protein from Moringa oleifera Lam" BIOCHIMICA ET BIOPHYSICA ACTA, AMSTERDAM, NL, vol. 1243, no. 3, 1995, pages 477-481, XP002245845 ISSN: 0006-3002
- BROIN M ET AL: "Flocculent activity of a recombinant protein from Moringa oleifera Lam. seeds." APPLIED MICROBIOLOGY AND BIOTECHNOLOGY, vol. 60, no. 1-2, October 2002 (2002-10), pages 114-119, XP002315280 ISSN: 0175-7598

### SEQUENCE LISTING

<110> OPTIMA ENVIRONNEMENT S.A.
   Mermod, Nicolas
   Suarez, Mougli
<120> Plant-derived peptides harboring water-cleaning and antimicrobial activities
<130> 159-16.WO
<160> 10
<170> PatentIn version 3.3
<210> 1
   <211> 21
   <212> PRT
   <213> Escherichia coli
<400> 1
   Pro Gln Arg Cys Pro Ser Leu Arg Gln Ala Val Gln Leu Thr His Gln
   1               5                   10                  15
   Gln Gln Arg Gln Val
               20
<210> 2
   <211> 31
   <212> PRT
   <213> Escherichia coli
<400> 2
   Arg Cys Gly Gln Gln Leu Arg Asn Ile Ser Pro Pro Gln Arg Cys Pro
   1               5                   10                  15
   Ser Leu Arg Gln Ala Val Gln Leu Thr His Gln Gln Gln Gly Gln
               20                  25                  30
<210> 3
   <211> 21
   <212> PRT
   <213> Escherichia coli
<400> 3
   Pro Gln Arg Cys Pro Ser Leu Arg Gln Ala Val Gln Leu Thr His Gln
   1               5                   10                  15
   Gln Gln Gly Gln Val
               20
<210> 4
   <211> 16
   <212> PRT
   <213> Escherichia coli
<400> 4
   Pro Gln Arg Cys Pro Ser Leu Arg Gln Ala Val Gln Leu Thr His Gln
   1               5                   10                  15
<210> 5
   <211> 22
   <212> PRT
   <213> Escherichia coli
<400> 5
   Gln Gly Pro Gly Arg Gln Pro Asp Phe Gln Arg Cys Gly Gln Gln Leu
   1 5 10 15
   Arg Asn Ile Ser Pro Pro
   20
<210> 6
   <211> 60
   <212> PRT
   <213> Escherichia coli
<400> 6
   Gln Gly Pro Gly Arg Gln Pro Asp Phe Gln Arg Cys Gly Gln Gln Leu
   1 5 10 15
   Arg Asn Ile Ser Pro Pro Gln Arg Cys Pro Ser Leu Arg Gln Ala Val
   20 25 30
   Gln Leu Thr His Gln Gln Gln Gly Gln Val Gly Pro Gln Gln Val Arg
   35 40 45
   Gln Met Tyr Arg Val Ala Ser Asn Ile Pro Ser Thr
   50 55 60
<210> 7
   <211> 21
   <212> PRT
   <213> Escherichia coli
<400> 7
   Gly Gln Val Gly Pro Gln Gln Val Arg Gln Met Tyr Arg Val Ala Ser
   1 5 10 15
   Asn Ile Pro Ser Thr
   20
<210> 8
   <211> 11
   <212> PRT
   <213> Escherichia coli
<400> 8
   Pro Gln Arg Cys Pro Ser Leu Arg Gln Ala Val
   1 5 10
<210> 9
   <211> 11
   <212> PRT
   <213> Escherichia coli
<400> 9
   Ser Leu Arg Gln Ala Val Gln Leu Thr His Gln
   1               5                   10
<210> 10
   <211> 12
   <212> PRT
   <213> Escherichia coli
<400> 10
   Ala Val Gln Leu Thr His Gln Gln Gln Gly Gln Val
   1               5                   10

## Description

### Field of the invention

The present invention relates to a family of proteins which may be used for different purposes such as coagulation agents for water treatment or as antimicrobial agents.

### State of the art

A protein corresponding to the above-cited definition, called FLO, is disclosed in PCT patent application WO 03/008441 A2 (OPTIMA ENVIRONNEMENT S.A.).

### Summary of the invention

The present invention concerns derivatives of FLO which, surprisingly, show similar or higher coagulating or antimicrobial activities than FLO.
The inventors have also unexpectedly found that some of those derivatives may show either a coagulating or an antimicrobial activity.
Finally, it was found that other FLO derivatives had neither a coagulating nor an antimicrobial activity.

### Detailed description of the invention

Table 1 summarizes the derivatives of FLO representing the object of the invention.
The following terms are used :
- +++: higher activity than FLO
- ++: equivalent activity of FLO
- +: lower activity than FLO
- -: no activity observed

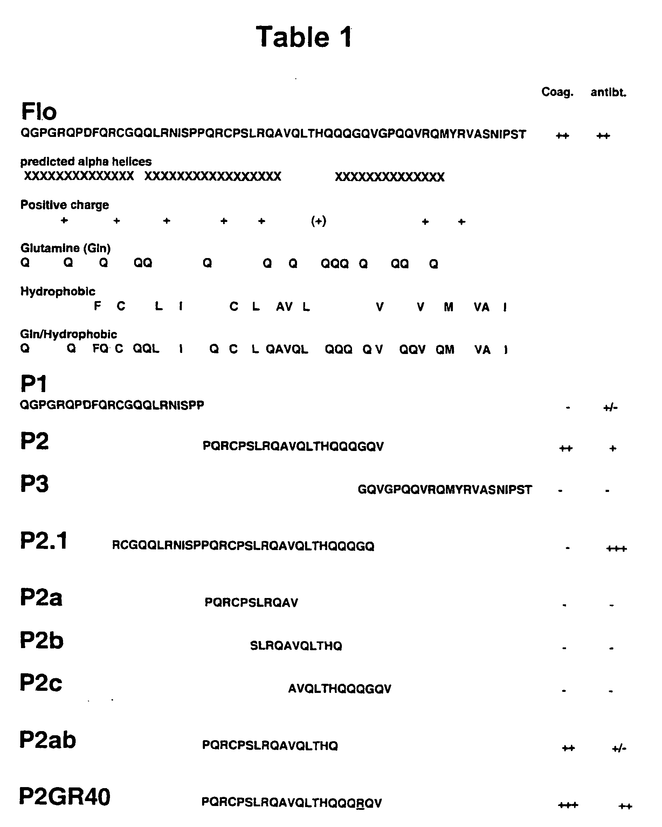

## Claims

1. A protein (P2GR40) defined by the sequence :
PQRCPSLRQAVQLTHQQQRQV.

2. Use of the protein of claim 1 as a coagulating agent.

3. Use of the protein of claim 1 as an antimicrobial agent.

4. A protein (P2.1) defined by the sequence :
RCGQQLRNISPPQRCPSLRQAVQLTHQQQGQ.

5. Use of the protein of claim 4 as an antimicrobial agent.

6. A protein (P2) defined by the sequence :
PQRCPSLRQAVQLTHQQQGQV.

7. Use of the protein of claim 6 as a coagulating agent.

8. Use of the protein of claim 6 as an antimicrobial agent.

9. A protein (P2ab) defined by the sequence :
PQRCPSLRQAVQLTHQ.

10. Use of the protein of claim 9 as a coagulating agent.

11. Use of the protein of claim 9 as a antimicrobial agent.

12. A protein (P1) defined by the sequence :
QGPGRQPDFQRCGQQLRNISPP.

13. Use of the protein of claim 12 as an antimicrobial agent.

## Patentansprüche

1. Protein (P20GR40), definiert durch die Sequenz:
PQRCPSLRQAVQLTHQQQRQV.

2. Verwendung des Proteins gemäß Anspruch 1 als Koagulationsmittel.

3. Verwendung des Proteins gemäß Anspruch 1 als antimikrobielles Mittel.

4. Protein (P2.1), definiert durch die Sequenz:
RCGQQLRNISPPQRCPSLRQAVQLTHQQQGQ.

5. Verwendung des Proteins gemäß Anspruch 4 als antimikrobielles Mittel.

6. Protein (P2), definiert durch die Sequenz:
PQRCPSLRQAVQLTHQQQGQV.

7. Verwendung des Proteins gemäß Anspruch 6 als Koagulationsmittel.

8. Verwendung des Proteins gemäß Anspruch 6 als antimikrobielles Mittel.

9. Protein (P2ab), definiert durch die Sequenz:
PQRCPSLRQAVQLTHQ.

10. Verwendung des Proteins gemäß Anspruch 9 als Koagulationsmittel.

11. Verwendung des Proteins gemäß Anspruch 9 als antimikrobielles Mittel.

12. Protein (P1), definiert durch die Sequenz:
QGPGRQPDFQRCGQQLRNISPP.

13. Verwendung des Proteins gemäß Anspruch 12 als antimikrobielles Mittel.

## Revendications

1. Protéine (P2GR40) définie par la séquence :
PQRCPSLRQAVQLTHQQQRQV.

2. Utilisation de la protéine selon la revendication 1 comme agent coagulant.

3. Utilisation de la protéine selon la revendication 1 comme agent antimicrobien.

4. Protéine (P2.1) définie par la séquence :
RCGQQLRNISPPQRCPSLRQAVQLTHQQQGQ.

5. Utilisation de la protéine selon la revendication 4 comme agent antimicrobien.

6. Protéine (P2) définie par la séquence :
PQRCPSLRQAVQLTHQQQGQV.

7. Utilisation de la protéine selon la revendication 6 comme agent coagulant.

8. Utilisation de la protéine selon la revendication 6 comme agent antimicrobien.

9. Protéine (P2ab) définie par la séquence :
PQRCPSLRQAVQLTHQ.

10. Utilisation de la protéine selon la revendication 9 comme agent coagulant.

11. Utilisation de la protéine selon la revendication 9 comme agent antimicrobien.

12. Protéine (P1) définie par la séquence :
QGPGRQPDFQRCGQQLRNISPP.

13. Utilisation de la protéine selon la revendication 12 comme agent antimicrobien.
